# EUROPEAN PATENT APPLICATION

(11) **EP 4 226 934 A1**
(43) Date of publication of application: **16.08.2023**
(21) Application number: 21880438.3
(22) Date of filing: 12.10.2021
(51) Int. Cl.: A61K 38/45, A61P 21/00, A23L 33/17, G01N 33/50

(54) **COMPOSITION FOR PREVENTING OR TREATING NEUROMUSCULAR DISEASE, COMPRISING PRMT1 PROTEIN OR GENE ENCODING SAME**

(30) Priority: 12.10.2020 KR 20200131378
(71) Applicant: Animuscure Inc., Suwon-si, Gyeonggi-do 16419 (KR)
(72) Inventor: KANG, Jong Sun, Suwon-si, Gyeonggi-do 16418 (KR); KIM, Hye Been, Suwon-si, Gyeonggi-do 16362 (KR); AN, Su Bin, Suwon-si, Gyeonggi-do 16362 (KR)
(74) Representative: Nederlandsch Octrooibureau
(86) International application number: PCT/KR2021/013971
(87) International publication number: WO 2022/080791

(57) **Abstract**

The present invention relates to a use for, by using the protein arginine methyltransferase 1 (PRMT1) protein or a gene encoding the same, preventing or treating a neuromuscular disease induced by motor neurons, particularly, damage to motor neurons caused by oxidative stress in the neuromuscular junction; and a method for screening a candidate material for activating the expression of PRMT1. PRMT1 deficiency in the motor nerve or neuromuscular junction induces aggravated degenerative motor nerve damage caused by aging, and DNA damage caused by oxidative stress and inflammation, thereby enabling the induction of neuromuscular disease, and thus the disease may be treated through the overexpression and activity of the PRMT1 protein and a gene encoding the same.

## Description

### [Technical Field]

The present invention relates to a use of a PRMT1 protein expressed in motor neurons and neuromuscular junctions, and a gene encoding the same for preventing or treating neuromuscular diseases.

### [Background Art]

A neuromuscular disease is a disease that affects peripheral nerves and muscles, and peripheral nerves refer to a nerve network that branches off the central nervous system and connects distant end organs such as muscles or skin to the central nervous system, and play a role in transmitting commands determined by the central nervous system to end organs such as muscles or transmitting sensory information, such as pain sensation, to the central nervous system.

A peripheral nervous system disease includes a disease of the peripheral nerve itself, dysfunction of the peripheral nerves caused by damage to the peripheral nerves due to a disease of the surrounding tissue adjacent to the peripheral nerves, and diseases which occur in neuromuscular junctions where the peripheral nerves are connected to the muscles, and muscles themselves. Representative clinical symptoms of the peripheral nervous system disease include motor nerve symptoms such as paralysis or muscle weakness, tingling or numbness in the hands or toes, pain, and the like.

A motor neuron disease induced by abnormalities in the peripheral nervous system as described above refers to a neurological disease in which muscle strength is normal, but motor neurons that control the activity thereof are damaged due to degenerative progression, and is caused by various factors such as genetic factors, environmental factors, toxin poisoning, viral infections, complications of other diseases, and aging. A motor neuron disease refers to unwanted movement or inability to perform desired movement due to muscular strength dysregulation. For example, Lou Gehrig's disease (ALS) is known as a representative motor neuron disease that slows down the movement of the limbs, and may gradually weaken pronunciation, swallowing, breathing, and the like, leading to death, and once the disease develops, the symptoms progress rapidly, and death may occur due to respiratory failure caused by respiratory muscle paralysis and other complications within 4 to 5 years. However, a fundamental treatment method for motor neuron diseases has not been developed to date, and only conservative measures such as alleviation of symptoms or reduction of pain through drugs or rehabilitation treatments, and the like are taken.

Further, with the aging of the population, the prevalence of degenerative motor neuron diseases, particularly caused by aging, is increasing, which may have a significant impact on human quality of life, so that there is a need for research on the fundamental cause and treatment method.

Protein arginine N-methyltransferase 1 (PRMT1) (Homo sapiens Gene ID: 3276; Mus musculus Gene ID: 15469) is one of the protein arginine methyltransferases, and although various functions have been recently studied, the mechanism at motor nerves or neuromuscular junctions is still not known.

### [Disclosure]

### [Technical Problem]

Thus, while studying various proteins and genes associated with damage to motor neurons, the present inventors found that, by performing research on a protein arginine methyltransferase 1 (PRMT1) gene expressed in motor neurons and neuromuscular junctions, it is possible to prevent damage to motor neuron cells due to cellular stress caused by various factors through the regulation of the gene, and achieve the effects of treating and alleviating a diseased caused by damage to motor neurons, thereby completing the present invention.

Therefore, an object of the present invention is to provide a composition for protecting motor neuron cells, comprising a PRMT1 protein or a gene encoding the same.

Another object of the present invention is to provide a composition for preventing, ameliorating or treating a neuromuscular disease, comprising a PRMT1 protein or a gene encoding the same.

Still another object of the present invention is to provide a method for screening a material for preventing, ameliorating or treating a neuromuscular disease in relation to whether PRMT1 is expressed.

### [Technical Solution]

To achieve the objects described above, the present invention provides a composition for protecting motor neuron cells, comprising a PRMT1 protein or a gene encoding the same.

The present invention also provides a method for protecting motor neuron cells, the method comprising: administering a PRMT1 protein or a gene encoding the same to a subject in need thereof.

The present invention also provides a use of a PRMT1 protein or a gene encoding the same for protecting motor neuron cells.

The present invention also provides a use of a PRMT1 protein or a gene encoding the same for preparing a drug for protecting motor neuron cells.

Furthermore, to achieve other objects of the present invention, the present invention provides a composition for preventing, ameliorating or treating a neuromuscular disease, comprising a PRMT1 protein or a gene encoding the same.

The present invention also provides a method for preventing, ameliorating or treating a neuromuscular disease, the method comprising: administering a PRMT1 protein or a gene encoding the same to a subject in need thereof.

The present invention also provides a use of a PRMT1 protein or a gene encoding the same for preventing, ameliorating or treating a neuromuscular disease.

The present invention also provides a use of a PRMT1 protein or a gene encoding the same for preparing a drug for preventing, ameliorating or treating a neuromuscular disease.

As an aspect of the present invention, the composition of the present invention has effects of protecting motor neurons and preventing, ameliorating or treating a neuromuscular disease, and thus, may be used for various purposes, such as a pharmaceutical, a health functional food, a functional food, an animal feed and a cell culture solution composition.

To achieve other objects of the present invention, the present invention provides a method for screening a therapeutic material of a neuromuscular disease, the method comprising: i) treating motor neuron cells with a candidate *in vitro*; ii) determining the expression or activity of PRMT1 in the motor neuron cells treated with the candidate; and iii) selecting candidates which enhance the expression or activity of PRMT1 compared to a non-treatment group as candidates for treating a neuromuscular disease.

Hereinafter, the present invention will be described in detail.

The present invention relates to a composition for protecting motor neuron cells, comprising a PRMT1 protein or a gene encoding the same.

The present invention also relates to a method for protecting motor neuron cells, the method comprising: administering a PRMT1 protein or a gene encoding the same to a subject in need thereof.

The present invention also relates to a use of a PRMT1 protein or a gene encoding the same for protecting motor neuron cells.

The present invention also relates to a use of a PRMT1 protein or a gene encoding the same for preparing a drug for protecting motor neuron cells.

The protection of the motor neuron cells of the present invention particularly includes protecting motor neurons from degenerative damage due to aging, but is not limited to, and is to improve a survival rate, maintain body weight, maintain behavioral ability, repair damaged nerves, repair DNA damage, and protect motor neuron cells from cellular stress, and refers to the alleviation or reduction of stress which may occur in motor neuron cells due to various factors such as aging.

As an aspect of the present invention, the protection of motor neurons is to prevent damage to motor neurons from aging, oxidative stress, and inflammatory stress, prevent muscular atrophy and fibrosis due to the damage to motor neurons, and reduce the stress sensitivity of motor neuron cells, and includes promoting the re-innervation of damaged motor neuron cells.

In addition, the present invention relates to a composition for preventing, ameliorating or treating a neuromuscular disease, comprising a PRMT1 protein or a gene encoding the same.

The present invention also relates to a method for preventing, ameliorating or treating a neuromuscular disease, the method comprising: administering a PRMT1 protein or a gene encoding the same to a subject in need thereof.

The present invention also relates to a use of a PRMT1 protein or a gene encoding the same for preventing, ameliorating or treating a neuromuscular disease.

The present invention also relates to a use of a PRMT1 protein or a gene encoding the same for preparing a drug for preventing, ameliorating or treating a neuromuscular disease.

The neuromuscular disease is caused by abnormalities in the peripheral nervous system, and the motor neuron disease includes muscular dystrophy, a motor neuron disease, myopathy, a neuromuscular junction disease, and the like as a neurological disease in which the motor neurons that control muscle movements are damaged due to degenerative progression. Furthermore, although not limited thereto, the neuromuscular diseases are classified as diseases of the neuromuscular junctions and muscles (disease classification code G70-G73), and includes myasthenia gravis, muscle nerve disorder, muscular dystrophy, myotonic disorder, myopathy, primary muscle disorder, myasthenic syndrome, Lambert-Eaton syndrome, and the like. Further, the motor neuron disease includes amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA), Huntington's disease, or the like, and includes other motor neuron diseases, and the like.

As an aspect of the present invention, the composition of the present invention may be used for various purposes such as a pharmaceutical, a health functional food, a functional food, an animal feed and a cell culture solution composition, and has an effect of preventing, ameliorating or treating the neuromuscular disease by preventing damage to motor neuron cells.

As used herein, the term "prevention" refers to all actions that suppress a neuromuscular disease or delay the onset of the neuromuscular disease by administering the pharmaceutical composition according to the present invention.

As used herein, the term "treatment" refers to all actions that ameliorate or beneficially change symptoms of a neuromuscular disease by administering the pharmaceutical composition according to the present invention.

As used herein, the term "subject" may refer to all animals comprising humans, in need of the motor neuron protection according to the present invention, or who have developed or are likely to develop a neuromuscular disease. The animal may be not only a human but also a mammal such as a cow, a horse, a sheep, a pig, a goat, a camel, an antelope, a dog, and a cat in need of treatment of similar symptoms.

The pharmaceutical composition according to the present invention may be used by being formulated in the form of an oral formulation, such as a powder, a granule, a tablet, a capsule, a suspension, an emulsion, a syrup, and an aerosol, an external preparation, a suppository, and a sterile injection solution, according to a typical method, and may further include a carrier, an excipient, or the like required for the formulation. Examples of pharmaceutically acceptable carriers, excipients or diluents which may be further included in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. When the pharmaceutical composition is formulated, the pharmaceutical composition is prepared using a diluent or excipient, such as a filler, an extender, a binder, a wetting agent, a disintegrant, and a surfactant, which are commonly used.

For example, a solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with the extract or compound. Further, in addition to a simple excipient, lubricants such as magnesium stearate and talc are also used. A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, a fragrance, a preservative, and the like.

Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerogelatin, and the like.

The pharmaceutical composition of the present invention may be orally administered or may be parenterally administered (applied intravenously, subcutaneously, intraperitoneally, or locally), and the administration dose may vary depending on a patient's condition and body weight, severity of disease, drug form, and administration route and period according to the target method, and the administration dose may be selected in an appropriate form by those skilled in the art.

The pharmaceutical composition of the present invention is administered in a pharmaceutically effective amount. In the present invention, the "pharmaceutically effective amount" refers to an amount sufficient to treat diseases as an amount applicable to medical treatment, and the criteria thereof may be determined according to types of diseases of patients, the severity of disease, the activity of drugs, sensitivity to drugs, administration time, administration route, excretion rate, treatment period, ingredients used in combination, and other items. The pharmaceutical composition of the present invention may be administered as an individual therapeutic agent or may be administered in combination with other therapeutic agents, and may be administered sequentially or simultaneously with therapeutic agents in the related art. The administration dose may be determined at a level that can minimize side effects in consideration of all of the above factors, and may be easily determined by those skilled in the art. Specifically, the administration dose of the pharmaceutical composition may vary depending on the patient's age, body weight, severity, sex, and the like, and generally, 0.001 to 150 mg of the pharmaceutical composition and more preferably, 0.01 to 100 mg of the pharmaceutical composition, per 1kg of the body weight, may be administered daily or every other day, or one to three times a day. However, this is exemplary, and the administration dose may be set differently, if necessary.

In addition, the composition of the present invention may be a food or health functional food, and in particular, the "health functional food" refers to a food manufactured and processed using raw materials or ingredients that have functional properties that are useful for the human body according to Health Functional Food Act No. 6727, and "functional" is meant to be taken for the purpose of regulating nutrients to the structure and function of the human body, or obtaining effects useful for public health use, such as physiological effects.

The food or health functional food of the present invention can be manufactured and processed to prevent and improve motor neuron diseases in a pharmaceutical administration form such as a powder, a granule, a tablet, a capsule, a pill, a suspension, an emulsion, and a syrup, or as a health functional food such as a tea bag, an infusion, a beverage, a candy, a jelly, and a gum.

The food or health functional food composition of the present invention may be used as a food additive, and may be commercialized alone or in combination with other ingredients. Furthermore, the food or health functional food composition of the present invention may include nutrients, vitamins, electrolytes, flavors, colorants, enhancers, pectic acids and salts thereof, alginic acid and salts thereof, organic acids, protective colloid thickeners, pH adjusters, stabilizers, preservatives, glycerin, alcohols, carbonating agents used in carbonated drinks, and the like. The ingredients may be used alone or in combination, and may be used in combination in suitable amounts.

Further, the present invention provides a method for screening a therapeutic material of a motor neuron disease, the method comprising: i) treating motor neuron cells with a candidate in vitro; ii) determining the expression or activity of PRMT1 in the motor neuron cells treated with the candidate; and iii) selecting candidates which enhance the expression or activity of PRMT1 compared to a non-treatment group as candidates for treating a motor neuron disease.

In the present invention, the determining of the expression or activity of PRMT1 may be performed by various methods capable of measuring the amount of RNA or protein produced by the expression of the PRMT1 gene. Preferably, the expression level of RNA was confirmed using real-time PCR (RT-PCR), a microarray, northern blotting, and the like, and protein activity and expression may be confirmed by a method such as immunoprecipitation, immunostaining, chromatin immunostaining, enzyme-linked immunosorbent assay (ELISA), and western blotting, and is not limited to that method.

### [Advantageous Effects]

The composition of the present invention includes a PRMT1 protein or a gene encoding the same, and the PRMT1 protein is a factor that plays an important role associated with the prevention of damage to motor neuron cells, and has effects of preventing damage to motor neurons from stress due to various factors, and preventing, ameliorating and treating motor neuron diseases, and the like due to the damage to motor neurons and degenerative factors.

### [Description of Drawings]

FIG. 1 illustrates the results of observing senescence-based survival rate in mice specifically lacking PRMT1 in motor neurons (PRMT1 mnKO).
FIG. 2 illustrates the results of confirming body weight loss due to aging in PRMT1 mnKO mice.
FIG. 3 shows the results of limp clasping in PRMT1 mnKO mice in order to observe changes in behavioral ability due to aging.
FIG. 4 shows the results of comparing the distance moved and speed of PRMT1 mnKO mice with those of the control in an open field test.
FIG. 5 shows the results of comparing the time that RMT1 mnKO mice endured in a rotarod test with that of the control.
FIG. 6 illustrates the results in which the hind leg muscle weight is recovered after damage to motor neurons in PRMT1 mnKO mice.
FIG. 7 shows the results of confirming that the muscle fiber size is significantly reduced in the TA muscle of PRMT1 mnKO mice.
FIG. 8 illustrates the results of confirming the degree of muscle fibrosis of PRMT1 mnKO mice.
FIG. 9 illustrates the results of comparing the expression degrees of nerve regeneration and muscle atrophy markers in PRMT1 mnKO mice with those in the control.
FIG. 10 shows the results of confirming the re-innervation degree of the axons in PRMT1 mnKO mice in comparison with the control.
FIG. 11 shows the results of confirming whether the stress-related sensitivity of PRMT1 mnKO mice is increased through the expression of DNA damage markers.
FIG. 12 shows the results of confirming, through western blot analysis, increases in expression level of stress-related proteins in a motor neuron cell line (NSC34) in which PRMT1 protein expression was suppressed.
FIG. 13 illustrates the results of confirming, by DHR123 staining, that ROS detection was increased in a motor neuron cell line (NSC34) in which PRMT1 protein expression was suppressed.
FIG. 14 confirms the result that the expression of a DNA damage-associated gene (γ-H2AX) was increased in a motor neuron cell line (NSC34) in which PRMT1 protein expression was suppressed.
FIG. 15 shows the results of confirming, through reduction of oxidative stress markers and inflammatory markers, the protective effect of neuronal cell stress induced by TNFα treatment in a motor neuron cell line (NSC34) in which PRMT1 protein was overexpressed.

### [Modes of the Invention]

Hereinafter, the present specification will be described in detail with reference to Examples in order to specifically explain the present specification. However, the Examples according to the present specification may be modified in various forms, and it is not interpreted that the scope of the present specification is limited to the Examples described below in detail. The Examples of the present specification are provided to more completely describe the present specification to a person with ordinary skill in the art.

### Example 1. Materials and experimental methods

### 1-1) Construction of experimental models

Constant conditions (temperature 22 ± 2 °C, humidity 55 ± 5%) were maintained in an experimental animal breeding room. A light cycle of 12 hours a day and a dark cycle of 12 hours a day were adapted.

Mice carrying a PRMT1-floxed allelic gene (PRMT1f/f purchased from EUCOMM) were crossed with transgenic mice expressing Cre under the control of an Hb9 promoter (Hb9-Cre provided by Jackson Laboratory). For sciatic nerve denervation surgery, the anesthetization of the animals was induced with 4% isoflurane and then maintained by continuous inhalation of 2% isoflurane on a surface heated to 37°C. The skin was incised posteriorly parallel to the left femur. The sciatic nerve was exposed to the mid-thigh and then damaged using tongs for 10 seconds. After denervation, the nerve was repositioned, the muscle was closed with surgical dissolvable sutures, and the skin incision was sutured with suture tape. Carprofen (5 mg/kg) was subcutaneously administered for postoperative pain control.

### 1-2) Behavior analysis

To analyze behavioral differences between PRMT1 f/f and PRMT1 hb9cre mice, open field and rotarod tests were performed.

The open field test was performed in an open plastic container (30 × 30 × 30 cm). When each experiment was initiated, mice were placed in the corners of the plastic container and movements were recorded for 20 minutes. The distance traveled and speed were analyzed through Noldus EthoVision 13.0 tracking software. Motor coordination and balance were measured using a rotarod apparatus (model 7600, Ugo Basile). For a fixed speed rotarod test (17 rpm), mice were disposed in a rotating cylinder after receiving 3 training sessions for 3 days before an actual experiment. A cut-off of 200 seconds per session was used. For an accelerated rotarod test (for 300 seconds, 4 to 40 rpm), mice received 3 training sessions for 3 days before an actual experiment. In both of the above two experiments, the time to fall off the rotarod was recorded.

### 1-3) Cell culture and transfection

NSC34 cells were cultured in a composition of 10% FBS, 1% GlutaMAX, 10 units/mL penicillin, and 10 µg/mL streptomycin based on DMEM media. NSC34 cells were transfected with a pcDNA, PRMT1-HA plasmid using a Mirus transfection reagent.

To make the expression of PRMT1 deficient in NSC 34 cells, the cells were transfected with Adenoviral pLKO.1-puro, PRMT1-shRNA.

### 1-4) Protein and RNA analysis

After cells were harvested, cells were lysed by adding a lysis buffer (20 mM Tris-HCl, pH8, 150 mM NaCl, 1% Triton X, proteinase inhibitor) for 30 minutes, then centrifuged at 13000 rpm for 30 minutes, and then a cell lysate sample was quantified, the same amount of protein was subjected to SDS-PAGE electrophoresis, and transferred to a PVDF membrane. After reaction with the corresponding primary and secondary antibodies, protein expression levels were confirmed by exposing the protein to X-ray film using an ECL reagent.

NSC34 cells and muscle tissue were homogenized with FastPrepR-24 (MP Biomedicals) and RNA was extracted using an easy-spin Total RNA Extraction Kit (iNtRON Biotechnology). Fold changes in gene expression were normalized compared to the expression of the ribosomal gene L32.

### 1-5) Histology and immunofluorescence analysis

The tibialis anterior muscle and extensor digitorum longus muscle were used as tissues used in the experiments, and the tibialis anterior muscle was frozen and fixed with OCT compound using liquefied nitrogen immediately after isolation, and then stored in an ultra-low temperature freezer at -80°C. Tissue sections were cut into a thickness of 7 µm using a cryomicrotome maintained at -20°C, and the degree of fibrosis was observed after Sirius Red staining.

Immunohistochemical staining was performed using the tibialis anterior muscle isolated from the animal tissues. The frozen sections stored in an ultra-low temperature freezer at -80°C were washed twice with 1×PBS. The frozen sections were fixed at ordinary temperature for 15 minutes using 4% paraformaldehyde and washed twice with 1×PBS. The tissue was reacted with a permeabilization buffer (0.5% Triton-X/PBS) at ordinary temperature for 5 minutes, and washed twice with 1xPBS. The tissue was reacted with a TE buffer at 100°C for 10 minutes for antigen retrieval, and washed twice with 1×PBS. After blocking with a blocking buffer (5% goat serum, 0.1% Triton-X) for 1 hour, a primary antibody (laminin) was diluted 1:500 in the blocking buffer and reacted at 4°C for 12 hours, and then washed twice with 1xPBS. After reacting with a secondary antibody, the tissue was washed twice with 1xPBS, encapsulated, and analyzed under a fluorescence microscope.

Further, neuromuscular junction staining (NMJ staining) was performed using the extensor digitorum longus muscle isolated from the animal tissue. The extensor digitorum longus muscle was isolated, immediately fixed using 4% paraformaldehyde at ordinary temperature for 15 minutes, and washed three times with 1xPBS. After blocking with a blocking buffer (3% BSA, 0.5% Triton-X) for 2 hours, a primary antibody (neurofilament, synaptophysin) was diluted 1:300 in the blocking buffer and reacted at 4°C for 24 hours, and then washed three times with 1xPBS. A secondary antibody and BTX antibody conjugated with the secondary antibody were reacted at room temperature for 2 hours, then washed twice with 1xPBS, and then encapsulated for analysis under a fluorescence microscope.

### 1-6) Statistical analysis

All values were expressed as mean ± SEM or SD. Statistical significance was calculated by a paired or unpaired two-tailed Student's t-test or analysis of variance (ANOVA) test followed by Tukey's test. Differences were considered significant at P<0.05.

### Experimental Example 1. Effect of motor neuron-specific PRMT1 deficiency

### 1-1) Effect of aging-based survival rate and weight loss in PRMT1-deficient mice

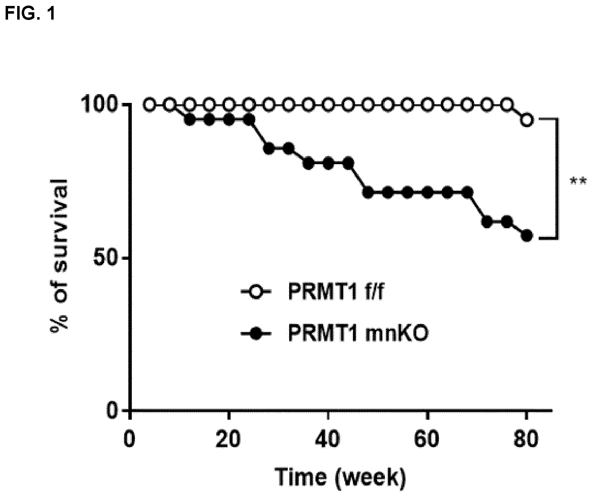

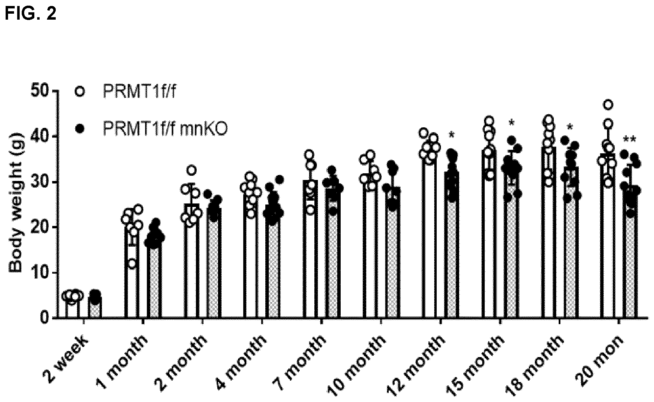

The survival rate and body weight of mice specifically lacking PRMT1 in motor neurons(PRMT1 mnKO) were measured at each month of age. As a result, it was confirmed that the survival rate and body weight of mice decreased sharply as aging progressed compared to wild-type mice (FIGS. 1 and 2). That is, it was confirmed that PRMT1, which is expressed in motor neurons, plays an important role in the maintenance of survival rate and body weight of aged mice.

### 1-2) Changes in behavioral ability due to aging in PRMT1-deficient mice

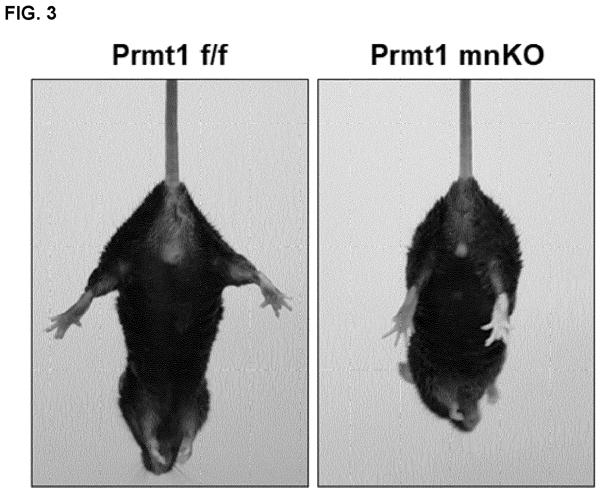

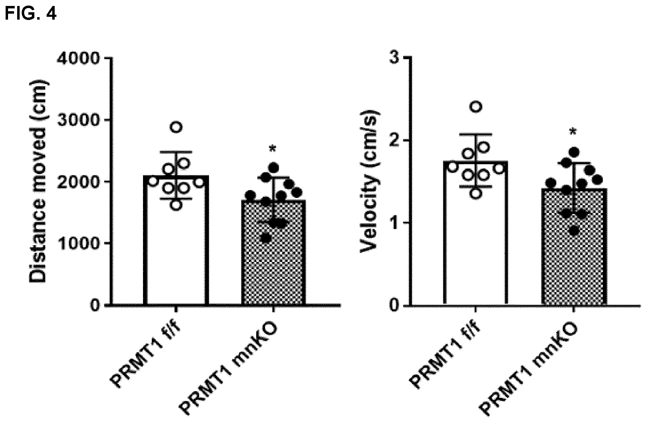

In the case of PRMT1 mnKO mice specifically lacking PRMT1 in motor neuron, as shown in FIG. 3, it could be confirmed that motor nerves were degenerated because a phenomenon of limp clasping was observed. In addition, as a result of confirming by performing an open field test as a behavioral evaluation, as shown in FIG. 4, it was confirmed that the moving distance and walking speed in the PRMT1 mnKO mice were decreased compared to normal mice (PRMT1 f/f).

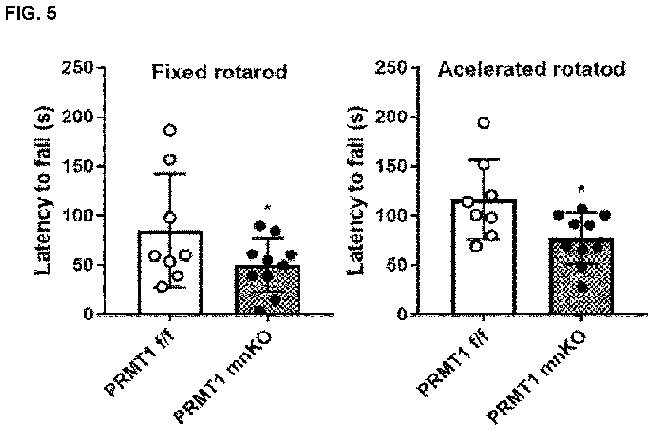

Furthermore, as a result of performing a rotarod test to evaluate the ability to regulate exercise capacity, as shown in FIG. 5, it was confirmed that PRMT1 mnKO mice had a shorter endurance time than PRMT1 f/f. From the experimental results described above, it can be seen that PRMT1 plays an important role in maintaining behavioral ability in aged mice in motor neurons.

### 1-3) Delayed nerve recovery in PRMT1-deficient mice

After sciatic nerve injury was induced in PRMT1 mnKO mice and normal mice (PRMT1 f/f), respectively, and re-innervation was induced for 28 days, the mice were sacrificed after behavioral analysis.

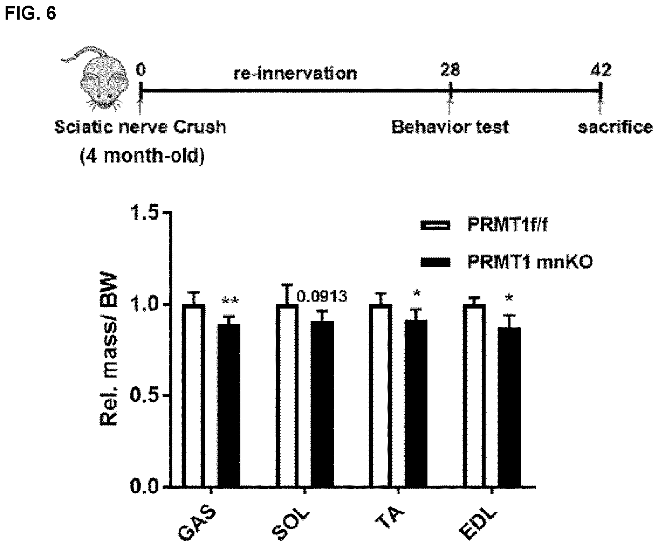

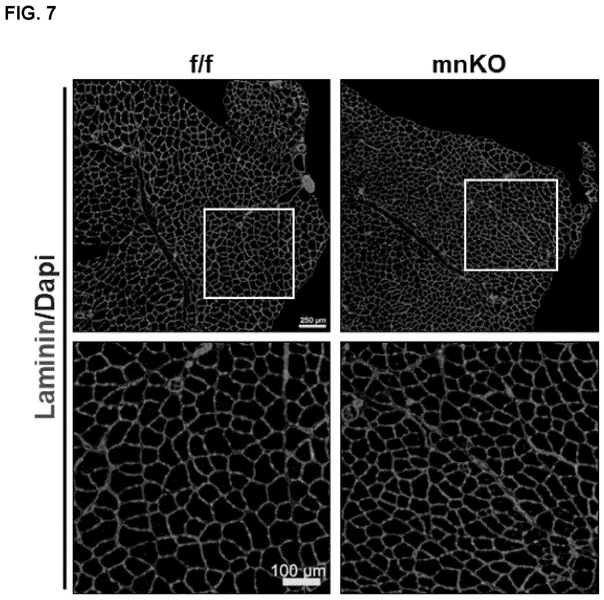

As a result of measuring and comparing the hindlimb muscle weights of PRMT1 mnKO mice and PRMT1 f/f, it was confirmed that the muscle recovery was delayed in PRMT1 mnKO mice (FIG. 6), and as a result of confirming the tibialis anterior (TA) muscle by laminin staining, it could be confirmed that the size of the myofiber of the PRMT1 mnKO mice was significantly decreased (FIG. 7).

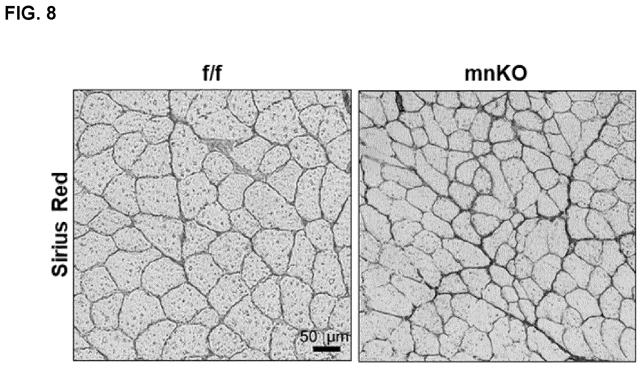

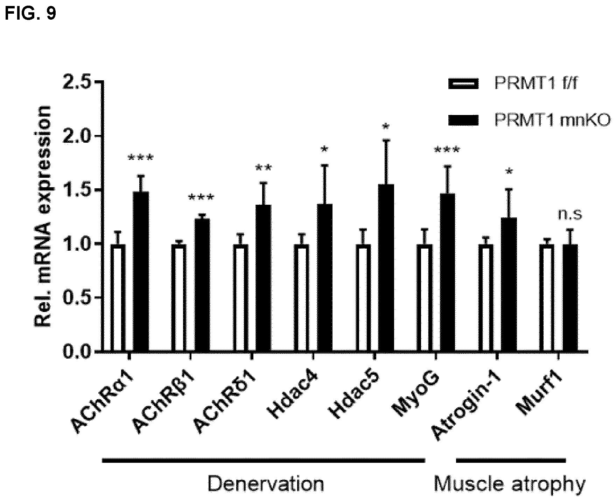

Furthermore, as a result of performing sirus red staining on muscle cells to confirm the degree of fibrosis, which is known to increase during muscle atrophy, it could be confirmed that the degree of fibrosis was increased in PRMT1 mnKO mice (FIG. 8), and in qPCR results for TA muscle, markers indicating denervation and muscle atrophy were increased compared to PRMT1 f/f mice (FIG. 9).

That is, from the above results, it can be seen that PRMT1 present in motor neurons plays an important role in the re-innervation of damaged motor neurons.

### 1-4) Delayed re-innervation of axons in PRMT1-deficient mice

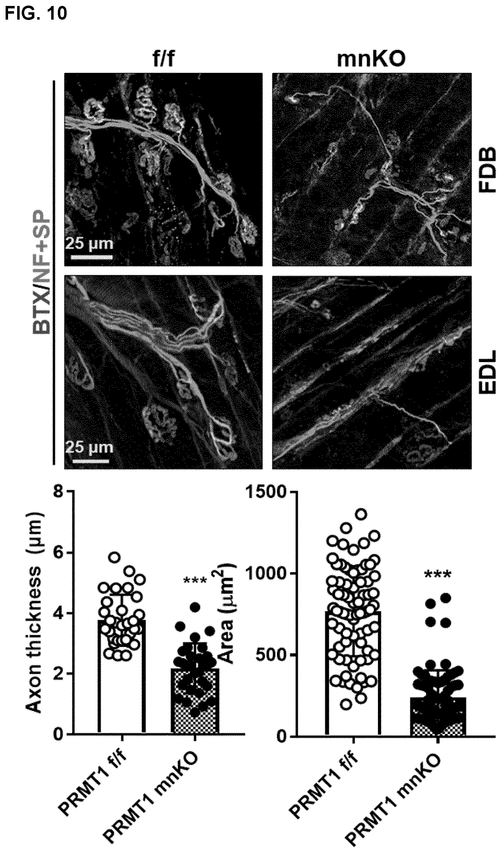

In neuromuscular junction staining (NMJ staining), it could be confirmed that in the flexor digitorum brevis (FDB) muscle and extensor digitorum longus (EDL) muscle, the thickness of motor neuron axons and the size of alpha_bungarotoxin (α-BTX), which is a neuromuscular junction marker, were thinner and smaller than those in PRMT1 f/f mice (FIG. 10). From this, it can be confirmed that re-innervation was delayed in the axons in the case of PRMT1 mnKO mice.

### 1-5) Increase in DNA damage and stress sensitivity in PRMT1-deficient mice

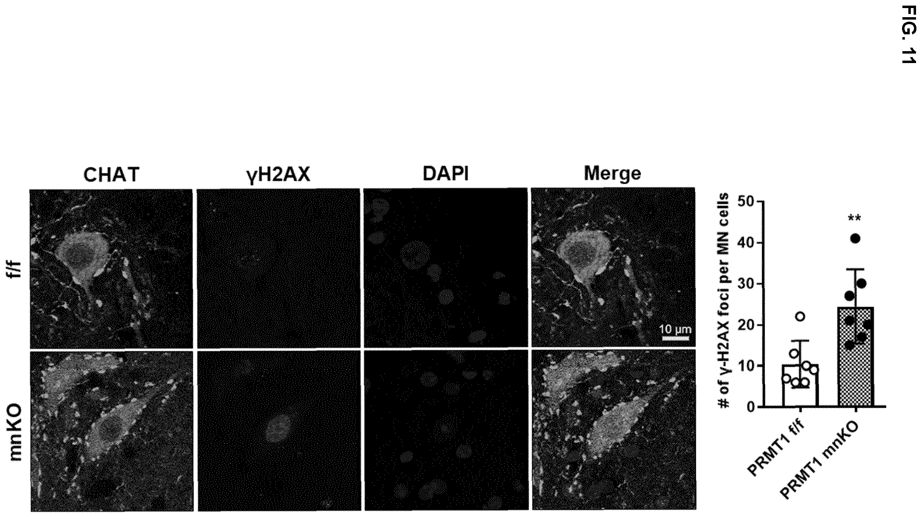

In order to confirm DNA damage and stress-related sensitivity in PRMT1 mnKO mice, the lumbar spinal cord, which is responsible for the movement of the hind leg muscles of mice, was serially sectioned and then observed after co-staining with choline acetyltransferase (ChAT), which is a motor neuron marker, and gamma H2AX. As a result, it could be confirmed that the expression of gamma H2AX, which is a DNA damage marker, was increased compared to PRMT1 f/f (FIG. 11). From the above results, it can be seen that PRMT1-deficient motor neurons have increased stress-related sensitivity.

### Experimental Example 2. Effect of PRMT1 expression in motor neuron cells

### 2-1) Effect of increase in cellular stress when PRMT1 expression is suppressed

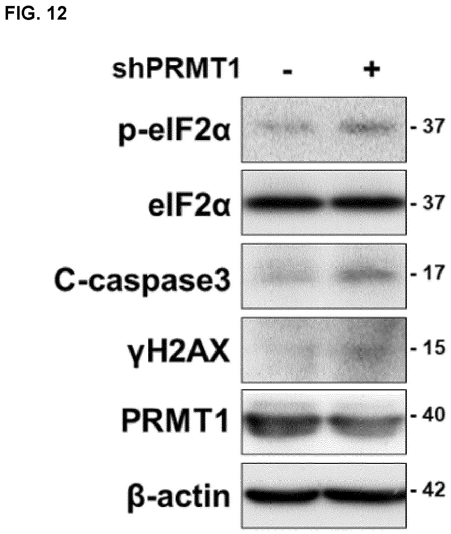

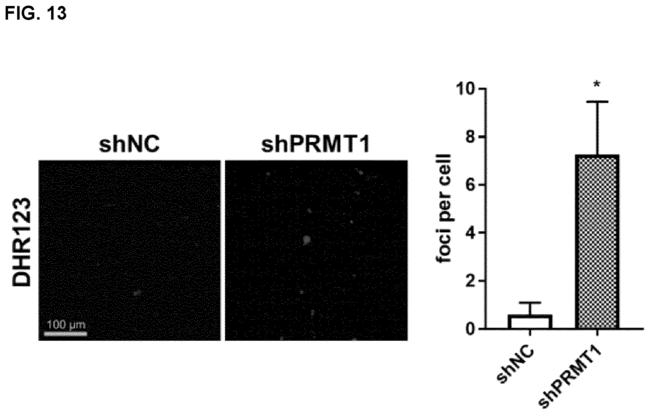

By inhibiting PRMT1 expression using an shPRMT1 adenovirus in an NSC34 cell line, which is a motor neuron, changes in the expression levels of cellular stress markers expressed in the cell line were confirmed. As a result, as shown in FIG. 12, it was confirmed, by western blotting analysis, that the expression of stress-related proteins p-eIF2a and c-caspase3 was significantly increased compared to the control, and the expression of gamma H2AX, which is a DNA damage protein, was also increased compared to the control. Further, through dihydrorhodamine123 (DHR123) staining, which is ROS staining, it was confirmed that the amount of ROS detected in NSC34 cells in which the expression of PRMT1 was inhibited was increased compared to the control (FIG. 13).

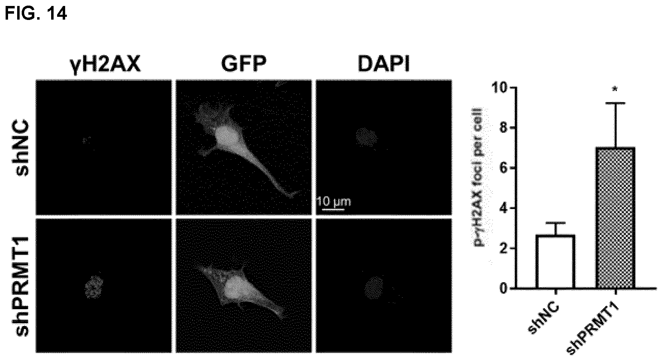

In addition, it was confirmed that when PRMT1 expression was inhibited using an shPRMT1 adenovirus in an NSC34 cell line, which is a motor neuron cell, and the cell line was stained with γ-H2AX, which is a DNA damage-related gene, γ-H2AX expression was significantly increased compared to the control (FIG. 14).

### 2-2) Effect of protecting neuron cells from cellular stress during overexpression of PRMT1

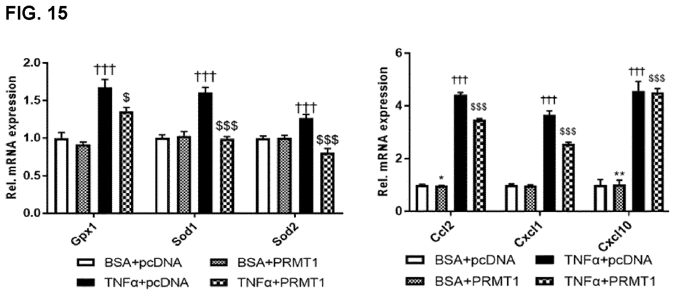

PRMT1 was overexpressed in an NSC34 cell line, which is a motor neuron cell, and mRNA expression of stress genes was observed by qPCR after TNFα treatment, which induces cell stress. It was confirmed that in TNFα-treated motor neuron cells, the expression levels of ROS stress markers (Gpx1, Sod1, and Sod2) and inflammatory stress markers (Ccl2, Cxcl1, and Cxcl10) were all increased, but all the markers were significantly decreased when PRMT1 was overexpressed (FIG. 15). From these results, it can be confirmed that overexpression of PRMT1 in motor neuron cells has an effect of being able to protect cell stress.

In the foregoing, the present invention has been examined mainly based on the preferred examples thereof. A person with ordinary skill in the art to which the present invention pertains will be able to understand that the present invention may be implemented in a modified form without departing from the essential characteristics of the present invention. Therefore, the disclosed examples should be considered not from a restrictive viewpoint, but from an explanatory viewpoint. The scope of the present invention is defined not in the above-described explanation, but in the claims, and it should be interpreted that all the differences within a range equivalent thereto are included in the present invention.

## Claims

1. A composition for protecting motor neuron cells, comprising a PRMT1 protein or a gene encoding the same.

2. The composition of claim 1, wherein the protection of motor neuron cells prevents damage to motor neurons due to aging, oxidative stress and inflammation.

3. The composition of claim 1, wherein the composition promotes the re-innervation of damaged motor neuron cells.

4. A pharmaceutical composition for preventing or treating a neuromuscular disease, comprising a PRMT1 protein or a gene encoding the same.

5. The composition of claim 4, wherein the neuromuscular disease is selected from the group comprising dystrophy, a motor neuron disease, myopathy, and a neuromuscular junction disease.

6. The composition of claim 5, wherein the motor neuron disease is selected from the group comprising amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA) and Huntington's disease.

7. A health functional food composition for preventing or ameliorating a neuromuscular disease, comprising a PRMT1 protein or a gene encoding the same.

8. The composition of claim 7, wherein the neuromuscular disease is selected from the group comprising dystrophy, a motor neuron disease, myopathy, and a neuromuscular junction disease.

9. The composition of claim 8, wherein the motor neuron disease is selected from the group comprising amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA) and Huntington's disease.

10. A method for screening a material preventing, ameliorating or treating a neuromuscular disease, the method comprising: i) treating motor neuron cells or neuromuscular junctions with a candidate; ii) determining the expression or activity of PRMT1 in the motor neuron cells or neuromuscular junctions treated with the candidate; and iii) selecting candidates which enhance the expression or activity of PRMT1 compared to a non-treatment group.
